Europäisches Patentamt

European Patent Office

Office européen des brevets

(11) Publication number: 0 263 608 A2

# (12) EUROPEAN PATENT APPLICATION

(21) Application number: 87308243.2

(22) Date of filing: 17.09.87

(51) Int. Cl.⁴: **C07K 15/08** , C07K 3/02 , A61K 37/02

(30) Priority: 18.09.86 US 908581
26.08.87 US 88432

(43) Date of publication of application:
13.04.88 Bulletin 88/15

(84) Designated Contracting States:
AT BE CH DE ES FR GB GR IT LI LU NL SE

(71) Applicant: THE CONTRIBUTORS TO THE
PENNSYLVANIA HOSPITAL
Eight & Spruce Streets
Philadelphia Pennsylvania(US)

(72) Inventor: Gasic, Gabriel J.
505 Hughes Road
Gulph Mills Pa. 19406(US)
Inventor: Gasic, Tatiana B.
505 Hughes Road
Gulph Mills Pa. 19406(US)
Inventor: Tuszynski, George P.
Box 377 R.D. 2
Mays landing NJ 08330(US)

(74) Representative: Bassett, Richard Simon
ERIC POTTER & CLARKSON 14 Oxford Street
Nottingham NG1 5BP(GB)

(54) Protein having anticoagulant and antimetastatic properties.

(57) The present invention comprises a biochemically pure protein, termed antistasin, which exhibits both antimetastatic and anticoagulant properties. Methods of producing the protein and methods of using the protein to inhibit or reduce metastasis and/or coagulation of blood are disclosed.

Antistasin has a MW of about 17,000 and an isoelectric point of 9.5. It is extracted from the salivary gland of the Mexican leech <u>Haementeria</u> officinalis by a process including heparin affinity chromatography. Antistasin has a partial amino acid sequence of:

...PHE-CYS-LYS-CYS-ARG-LEU-GLU-PRO-MET-LYS-ALA-THR-CYS-ASP-ILE-SER-GLU-CYS-PRO-GLU...;

or conservative substitutions thereof.

FIG. I
HPLC OF ANTISTASIN

## PROTEIN HAVING ANTICOAGULANT AND ANTIMETASTATIC PROPERTIES

### Field of the Invention

The present invention pertains to a method of preparing and using a biochemically pure protein or proteins, each having anticoagulant and antimetastatic properties, and to the protein(s) itself.

### Background of the Invention

As disclosed in U. S. Patent 4,588,587, of common assignment herewith, the saliva of blood sucking animals such as leeches, possesses anticoagulant activity. As also discussed in the above-referenced patent, an extract of certain leech salivary glands also exhibits antimetastatic effects. It has been postulated that these effects may result from a unique and complex combination of anticoagulants, and/or protease inhibitors, and/or other constituents of the extract which act synergistically.

The theoretical mechanism by which metastasis occurs is believed to involve several steps, such as entry of tumor cells into the organisms circulation (intravasculation); their transport by the blood stream; interactions of circulating malignant cells with platelets and plasma clotting factors with activation of the hemostatic system; interaction of the same cells with host cells other than platelets; arrest of tumor cells, surrounded by platelet aggregates and fibrin clots within capillaries; proteolytic attack of the blood vessel wall, particularly of its basement membrane, by tumor enzymes; escape from the circulation system (extravasculation); and formation of secondary tumors or colonies.

Crucial to this mechanism is the ability of the circulating tumor cell to initiate the formation of a blood clot which serves to lock the cell in a capillary. Falanga, et al (Biochemistry 24:5558-5567, 1985) isolated and characterized a particular cancer procoagulant which was shown to initiate coagulation through activation of factor X. In addition, other researchers cited in that reference have isolated cancer procoagulants which directly activate factor X. Finally, one of the present inventors' prior publications (Cancer Metastasis Reviews 3, 99-116 (1984) presents a summary of research into the activation of the coagulation system by tumor cells, which also discusses the possible involvement of factor X.

### Brief Description of the Invention

The present invention comprises a newly isolated, biochemically pure protein (or proteins) having antimetastatic and anticoagulant properties. This protein is derived from a leech salivary gland, specifically that of the Mexican leech, Haementeria officinalis. The invention also comprises a method of isolating the protein, and a method of using the protein to prevent or delay blood coagulation and metastasis. The protein, termed antistasin, is believed to inhibit coagulation of blood by inhibiting coagulation factor Xa but not thrombin.

Antistasin is isolated from an extract of salivary glands of leeches, particularly the Mexican leech Haementeria officinalis, by heparin affinity chromatography followed by anion exchange chromatography. The isolated antistasin has a molecular weight of approximately 17,000 under nondenaturing conditions. The protein has an isoelectric point of approximately 9.5 and is extremely active as an inhibitor of coagulation in in vitro analysis. Furthermore, the extract has been shown to be useful in inhibiting metastasis in in vivo tests using mice.

### Brief Description of Figures

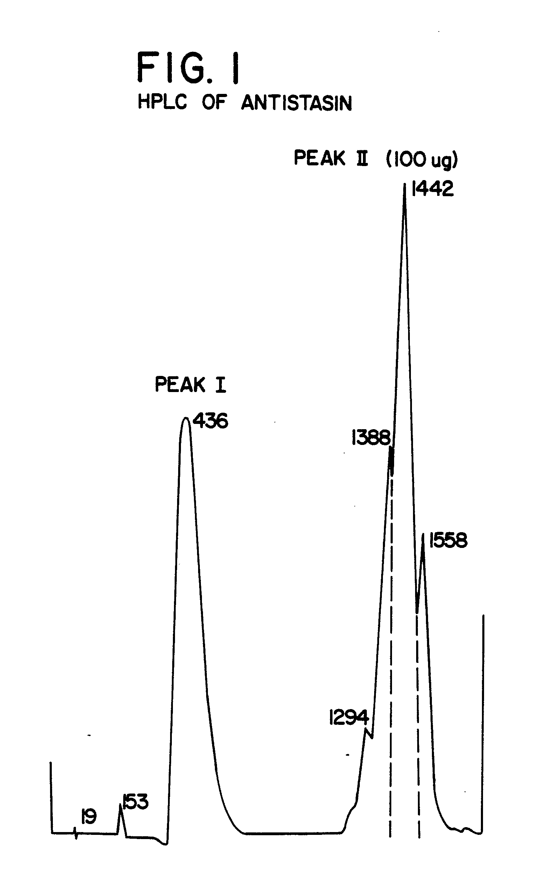

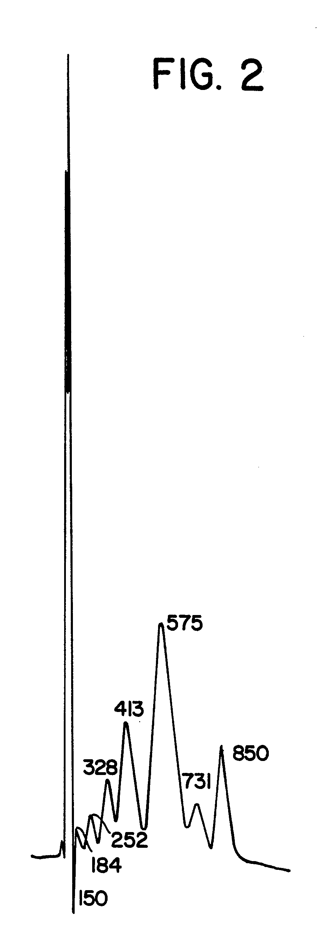

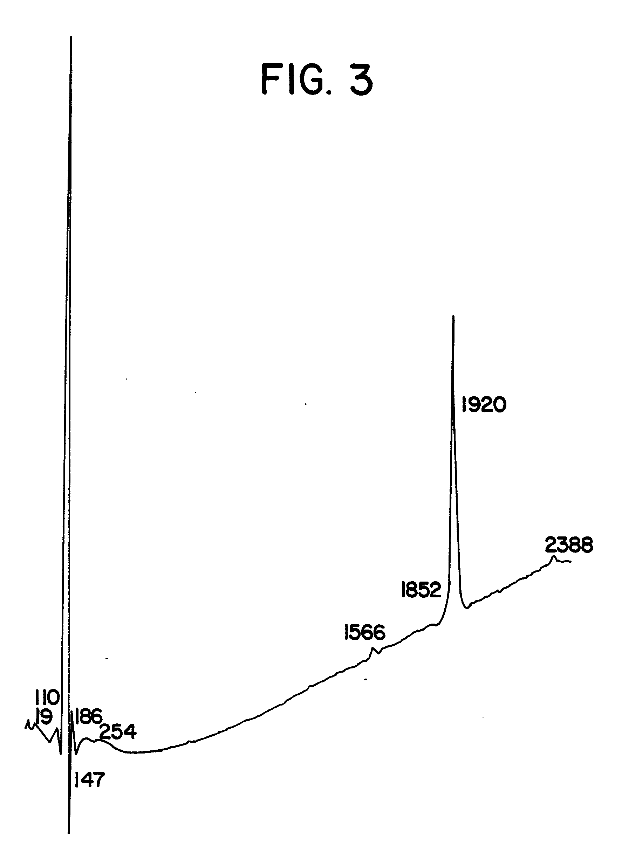

Figures 1, 2 and 3 are each curves showing the absorption spectrum, at 210 nm, of fractions eluted from a high pressure liquid chromatographic column, as further described in the detailed description. Numbers adjacent the peaks in the Figures indicate retention time in $10^{-2}$ minutes.

## Detailed Description of Invention

Antistasin is a biochemically pure protein or proteins, each of which is separated from a leech salivary gland extract by chromatography and selection of chromatographic fraction having anticoagulation activity. The product antistasin(s) are characterized by a selective capacity to inactivate factor Xa, but not thrombin.

Antistasin is purified to two different stages, Stage 1 having partial purity, and Stage 2 of apparent homogeneity.

Preliminary data on sequencing proteolytic digests of the apparently homogeneous preparation (Stage 2) suggest the possibility of natural homologues of antistasin. This invention encompasses all such homologues, provided that each one has inhibitory activity against Factor Xa.

It will be understood that other variants of any of the antistasins of the present invention are included, especially any variants that differ from the isolated antistasins only by conservative amino acid substitution. All such conservative amino acid substitutions are defined as "sets" in Table 1 of Taylor, W. R., J. Mol. Biol. 188, 233 (1986). Antistasin or fragments thereof in this application includes any such variations in the amino acid sequence, whether by conservative amino acid substitution, deletion, or other process, provided that the antistasin, after purification, is immunochemically reactive with antibodies specific for antistasin isolated from the Mexican leech, Haementeria officinalis.

Following is an example of this separation and selection method through the first stage of purification.

## Method of Making Antistasin (Stage 1)

An extract of the salivary gland of the Mexican leech is first prepared by extracting the dissected anterior and posterior salivary glands at 4°C by sonication in a buffer of 20mM HEPES (4-(2-hydroxyethyl)-1-piperazine ethane sulfonic acid), pH 7.8 containing 10 mN CaCl₂. The sonicated material is centrifuged at 8500g for 20 minutes and the supernatants pooled and centrifuged at 100,000g for 1 hour. The resulting supernatant protein suspension is frozen at -70° until needed. The antistasin is purified from the salivary gland extract by a two step chromatographic procedure using prolongation of the prothrombin time as an indicator of the presence of anticoagulant/antimetastatic activity.

The salivary gland extract is first placed on a heparin-agarose column equilibrated with 20 mM Tris-HCl, pH 8.7. Loading, washing, and elution of the heparin-agarose column are facilitated by the use of a gradient maker and liquid chromatography controller. Protein peaks eluting from the column are monitored by means of a UV monitoring system. The proteins not adhering to the column, and those which elute on wash with Tris buffer until no more adsorbance at 280 nM is observable are discarded. The washed column is then eluted by a combination of linear and step gradients using Tris buffer and Tris buffer containing 1 M NaCl. When protein peaks begin eluting, the gradient is held manually and proteins are allowed to elute from the column under isocratic conditions. After each protein peak elutes, the gradient is restarted.

The column fractions containing anticoagulant activity, as determined by assay for anticoagulation activity, are thereafter pooled, concentrated by centrifugation through ultrafiltration filters having molecular weight exclusion limits of 12,000, desalted on small columns of G-25 Sephadex and applied to a mono Q column equilibrated in Tris buffer. The mono Q column is then washed and eluted in a manner similar to that of the heparin-agarose column, yielding antistasin (Stage 1).

The partially purified protein derived in this manner is concentrated to 1 mg/ml and stored frozen at -70 degrees in Tris buffer. Further purification and analysis to Stage 2 grade purity is described in the Example, Figures 1-3 and Table 5.

## Characteristics of Antistasin

The molecular weight of the antistasin has been estimated by gel filtration on a fast protein liquid chromatograph and by SDS-gel electrophoresis. These analyses indicate a molecular weight of 17,000.

The isoelectric point of the antistasin was measured by chromatofocusing on a mono P column using the fast protein liquid chromatograph. This method yielded a pI of 9.5 for the material.

Amino acid analysis of the partially purified antistasin (Stage 1) was conducted on a Glenco MM-70 amino acid analyzer. The antistasin material was hydrolyzed with 6 NHCl for 24 hours. The resulting amino acid composition is presented as Table 1. Slightly different results are obtained with greater apparent protein purity, see Table 5. Another analysis of the Stage 1 purified product, indicated a somewhat different amino acid content, as shown in Table 1A. This may indicate the possible presence of somewhat different forms of antistasin in the Stage 1 product, or contaminants or derivatives thereof.

## Measurement of Antimetastatic Properties

Inhibition of experimentally induced metastasis was determined by injecting antistasin or a buffer control two hours prior to injection of $10^5$ T241 tumor cells followed by innoculation of similar quantities of antistasin two and four hous respectively after injection of the tumor cells.

After 14-19 days, the animals were sacrificed and the lung tumor colonies counted. Results are presented in Table 2.

## Anticoagulant Effects

One-stage clotting assays for measurement of the effect of antistasin on the prothrombin time, activated partial thromboplastin time, thrombin time, and factor X assay were performed at 37° in a final volume of 0.3 ml with the aid of a fibrometer. For the determination of the effect of antistasin on the prothrombin time, 100 $\mu$l of a saline dilution of protein followed by 100 $\mu$l of thromboplastin reagent containing 0.025 M CaCl$_2$ was added sequentially to 100 $\mu$l of normal human plasma and the clotting time determined.

For measurement of activated partial thromboplastin time, kaolin-cephalin reagent containing 0.025 M CaCl$_2$ was substituted for the thromboplastin reagent.

For measurement of the effect of antistasin on the thrombin time, the substrate solution was either 100 $\mu$l of normal human plasma or a 100 $\mu$l solution of a 1 mg/ml of fibrinogen in HEPES buffered saline and was clotted by the addition of 200 $\mu$l of a 0.30 unit/ml HEPES buffered saline solution of thrombin containing various amounts of purified antistasin. To maximise the effect on thrombin, 10 $\mu$l volumes of protein and thrombin were first incubated together for 5 minutes at 37 degrees before dilution and addition to the normal human plasma or fibrinogen.

For determination of the effect of antistasin on factor Xa activity, 100 $\mu$l of factor X deficient plasma was treated with 100 $\mu$l of 0.025 M CaCl$_2$ followed by 100 $\mu$l of purified factor Xa or 100 $\mu$l of purified factor Xa containing various amounts of antistasin.

The results of the above-described assays are presented in Tables 3 and 4.

The analyses conducted indicate that antistasin is a protein having a molecular weight of approximately 17,000 in a single chain with an isoelectric point of 9.5. Antistasin is a potent anticoagulant of both human and mouse plasma. As demonstrated in the various clotting time assays, antistasin acts through inhibition of the factor Xa pathway, rather than through interference with thrombin. Interestingly, the anticoagulant activity of antistasin appears more pronounced in vitro than in vivo. However, possible interferences have not been fully examined at this time.

## Statement Of Industrial Utility

The protein or proteins of the present invention are useful for the treatment of mammals to prevent or reduce coagulation of blood or metastasis. Particular applicability encompasses prophylactic treatment for thrombosis related diseases.

## EXAMPLE

4

## A. Purification to Stage 2

Antistasin was partially purified according to the method of Tuszynski, G.P. et al., J. Biol. Chem. 262 , 9718-9723 (1987). The partially purified material was further purified utilizing reverse phase HPLC on an acetonitrile/water gradient (see Figure 1). Factor Xa inhibitory activity appears in peak II, exclusively. Peak II contained a number of protein species and was further purified by reverse-phase HPLC utilizing an isopropanol/water gradient as shown in Figure 2. The fifth peak (denoted with an asterisk [*]) contained the highest Factor Xa inhibitory activity. This material was purified to apparent homogenity as shown in Figure 3, by HPLC utilizing an acetonitrile/water gradient, yielding antistasin (Stage 2).

## B. Structural Analysis

The amino acid composition of antistasin (Stage 2) is shown in Table 5. The antistasin material was hydrolyzed with 6 N HCL for 24 hours and analyzed on a Beckman Amino Acid Analyzer.

Antistasin (Stage 2) was subjected to amino acid sequence analysis in an Applied Biosystems Gas Phase Sequencer. The intact protein was blocked at its amino terminus rendering it resistant to sequence analysis. Accordingly, it was reduced and carboxymethylated to modify cysteine residues and finally cleaved with the proteolytic enzyme, V8 protease, which cleaves at glutamic acid residues. Approximately 10 peptide fragments of antistasin (Stage 2) were produced by this cleavage reaction and purified by reverse phase HPLC utilizing an acetonitrile/water gradient. The amino acid sequence of one of these fragments is:

PHE-CYS-LYS-CYS-ARG-LEU-GLU-PRO-MET-LYS-ALA-THR-CYS-ASP-ILE-SER-GLU-CYS-PRO-GLU.

Little contamination with other sequences was found for this particular fragment. However, an unconfirmed result was the presence of homologous sequences in other fragments, i.e., conservative substitutions elsewhere in the antistasin sequence.

Table 1

Amino Acid Composition of the Antistasin, Stage 1 Purity.[a]

| Amino Acid | nmoles | Ratio | # of Residues |
|---|---|---|---|
| Asx | 1.74 | 9.2 | 9 |
| Thr | 0.80 | 4.2 | 4 |
| Ser | 1.01 | 5.3 | 5 |
| Glx | 1.82 | 9.6 | 10 |
| Pro | ND[b] | | |
| Gly | 1.87 | 9.8 | 10 |
| Ala | 0.53 | 2.8 | 3 |
| Cys | 1.51 | 8.0 | 8 |
| Val | 0.46 | 2.4 | 2 |
| Met | 0.36 | 1.9 | 2 |
| Ile | 0.61 | 3.2 | 3 |
| Leu | 0.85 | 4.5 | 5 |
| Tyr | 0.34 | 1.8 | 2 |
| Phe | 0.48 | 2.5 | 3 |
| His | 0.19 | 1.0 | 1 |
| Lys | 1.44 | 7.6 | 8 |
| Arg | 1.40 | 7.4 | 7 |

[a]Average of two determination, expressed as nmole per 28 μg of protein.
[b] ND, not determined

## Table 1A

Amino Acid Composition of Antistasin, Stage 1 Purity, Second
Analysis [a]

| Amino Acid | % of Residues Per 17,000 Mr |
|---|---|
| Asx | 11.1 |
| Thr | 8.9 |
| Ser | 8.7 |
| Glx | 13.5 |
| Pro | 12.2 |
| Gly | 17.4 |
| Ala | 6.5 |
| Cys | ND |
| Val | 4.3 |
| Met | 2.2 |
| Ile | 4.3 |
| Leu | 6.3 |
| Tyr | 29.0 |
| Phe | 4.5 |
| His | 3.3 |
| Lys | 3.9 |
| Arg | 12.6 |

[a] 464 ng of protein was analyzed
[b] ND, not determined

Table 2

## % Inhibition of Lung Tumor Colonies by Antistasin, Stage 1 Purity

| Sample | Amount Injected (ug/mouse) | Number Of Mice | % Inhibition[a] |
|--------|------|------|------|
| | | *Experiment 1* | |
| SGE[b] | 1000 | 5 | 100 |
| Antistasin | 15 | 4 | 85 |
| | | *Experiment 2* | |
| SGE | 1000 | 4 | 89 |
| Antistasin | 62 | 4 | 100 |
| | | *Experiment 3* | |
| SGE | 1000 | 7 | 97 |
| Antistasin | 73 | 7 | 93 |

[a] % inhibition is calculated as follows: median number of lung tumors of control animals minus nedian number of lung tumors of treated animals divided by the median number of lung tumors from control animals times 100. The median number of lung tumors of control animals ranged from 20-60 tumors per animal. That of treated animals ranged from 0-5 tumors per animal.

[b] SGE = crude Leech Salivary Gland Extract

8

Table 3

The Effect of Antistasin on the Prothrombin and
Activated Partial Thromboplastin Times

| Addition | Clotting Time (sec)[a] | Difference[b] |
|---|---|---|
| Buffer | 95.6, 12.8 | — — |
| Antistasin[c] | 152.1, 82.8 | 56.5, 70.0 |

[a]Each value is the average of two determinations. Clotting times were obtained with normal human pooled plasma. The first clotting time is the activated partial thromboplastin time and the second time is the prothrombin time.
[b]Difference is protein minus buffer control. The first value is that calculated for the activated partial thromboplastin time and the second value is that for the prothrombin time.
[c]Two μg of protein, Stage 1 purity, were added to the test plasma.

Table 4

The Effect of Antistasin, Stage 1 on the Clotting Time of
Thrombin and Factor Xa

| Protein (ug) | Clotting Time (sec) | Protein (ug) | Clotting Time (sec) |
|---|---|---|---|
| Thrombin | | Factor Xa | |
| 0 | 33.9, 22.2[a] | 0 | 25.9 |
| 2.42 | 31.4, 20.6[a] | 1.21 | 275.9 |
| | | 2.42 | 1369.5 |

[a]The first clotting time was obtained using 1 mg/ml fibrinogen as substrate and the second was obtained using normal human plasma as substrate. Factor Xa clotting times were obtained using human factor X deficient plasma. Clotting times are an average of two determinations. Thrombin and factor Xa present in the assay mixtures were 0.30 and 0.10 units, respectively.

## TABLE 5

Amino Acid Composition of Antistasin, Stage 2 purity,
Mr – 17,000

| Amino Acid | Residues per Mr – 17,000 |
|---|---|
| Asp | 11 |
| Thr | 5 |
| Ser | 8 |
| Glu | 14 |
| Pro | 10 |
| Gly | 13 |
| Ala | 4 |
| Val | 2 |
| Ile | 5 |
| Leu | 7 |
| Tyr | 2 |
| Phe | 3 |
| Lys | 11 |
| His | 2 |
| Arg | 15 |
| Met | 3 |
| Cys | 20 |

**Claims**

1. A biochemically pure protein (antistasin) having a molecular weight of about 17,000 and an isoelectric point of 9.5, and a capacity to inhibit or delay blood coagulation by selective inhibition by selective inhibition of coagulation factor Xa.

2. A protein of Claim 1 wherein said protein is derived from the salivary gland of the leech Haementeria officinalis.

3. A purified protein of Claim 1 or 2 wherein said protein possesses antimetastatic properties.

4. A process for the production of a protein derived from the salivary gland of the leech Haementeria officinalis, said process comprising the steps of:

a) dissecting and removing the salivary gland tissue of said leech;

b) homogenizing and solubilizing said salivary gland tissue in an aqueous solution comprising a buffered salt having a fixed pH of 7.8 to produce a homogenate; and

c) centrifuging said homogenate to produce supernatant protein suspension fractions, and by assaying said supernatant fractions and selecting a product fraction characterized by its inhibition of coagulation factor Xa, said supernatant fraction being separated at a force of at least 100,000g for a period of at least 1 hour, and said 100,000g supernatant fraction is further treated by the steps of

d) placing said supernatant fraction in contact with a column of heparin agarose equilibrated with Tris buffer, pH 8.7;

e) separating fractions containing proteins which are not adsorbed on said column from fractions containing proteins which are adsorbed on said column; and

f) selecting from said adsorbed fractions, a protein having anticoagulant properties.

5. The process of Claim 4 further including the steps of:

g) eluting a first portion of said adsorbed fractions using a buffered salt solution until spectrophotometric absorbance at 280nM is no longer detected;

h) eluting a second portion of said adsorbed fractions using a gradient elution liquid comprising 20mM Tris buffer and 1M NaCl in Tris buffer; and testing the eluted fractions to identify an eluate fraction possessing anticoagulant activity.

6. A process, as recited in Claim 5, further including:

i) concentrating said eluate frations having anticoagulant acitivity by centrifugation through ultra-filters having molecular weight exclusion limits of about 12,000;

j) de-salting said concentrated eluate fraction;

k) adsorbing said de-salted concentrated eluate fraction on a Mono-Q anion exchange column; and

l) eluting said adsorbed fraction with NaCl step gradient in the range 0.10-0.30M in Tris buffer solution to yield antistasin.

7. The product of Claim 4 wherein said product fraction is characterized by in vitro inhibition activity for coagulation factor Xa.

8. The product of Claim 4 wherein said product fraction antistasin is characterized by antimetastatic properties.

9. The protein of any of Claims 1 to 3 for use in the prevention of metastasis or prolonging of blood clotting time in a mammal.

10. Antistasin polypeptides comprising one or more polypeptides, each of which inhibit or delay blood coagulation by selective inhibition of coagulation factor Xa, said polypeptides each having a molecular weight of about 17,000, an isoelectric point of about 9.5, and a partial amino acid sequence of:

...PHE-CYS-LYS-CYS-ARG-LEU-GLU-PRO-MET-LYS-ALA-THR-CYS-ASP-ILE-SER-GLU-CYS-PRO-GLU...; or conservative substitutions thereof.

11. A fragment of any of the antistasin polypeptides, said fragment consisting essentially of oligopeptides or small peptides with sequences of 5 or more amino acids, said sequences being those of the polypeptides of Claim 10.

12. A therapeutic composition for the treatment or prophylaxis of thrombosis-related diseases, comprising an effective amount of antistasin polypeptides of Claim 10, or fragments thereof according to Claim 11.

# FIG. I
## HPLC OF ANTISTASIN

FIG. 2

FIG. 3